(19) Europäisches Patentamt
European Patent Office
Office européen des brevets

(11) Veröffentlichungsnummer: **0 158 954**
**A2**

(12) **EUROPÄISCHE PATENTANMELDUNG**

(21) Anmeldenummer: **85104250.7**

(22) Anmeldetag: **09.04.85**

(51) Int. Cl.[4]: **C 07 D 401/06**
C 07 D 215/08, C 07 D 215/18
A 01 N 47/38

(30) Priorität: **20.04.84 JP 78719/84**

(43) Veröffentlichungstag der Anmeldung:
**23.10.85 Patentblatt 85/43**

(84) Benannte Vertragsstaaten:
**AT BE CH DE FR GB IT LI NL**

(71) Anmelder: **NIHON TOKUSHU NOYAKU SEIZO K.K.**
**No.4, 2-chome, Nihonbashi Honcho Chuo-ku**
**Tokyo 103(JP)**

(72) Erfinder: **Kurahashi, Yoshio**
**47-15, Ohya-machi**
**Hachioji-shi Tokyo(JP)**

(72) Erfinder: **Shiokawa, Kozo**
**210-6, Shukugawara Tama-ku**
**Kawasaki-shi Kanagawa-ken(JP)**

(72) Erfinder: **Goto, Toshio**
**3454-21, Honmachida**
**Machida-shi Tokyo(JP)**

(72) Erfinder: **Kagabu, Shinzo**
**432-131-105, Terada-machi**
**Hachioji-shi Tokyo(JP)**

(72) Erfinder: **Kamochi, Atsumi**
**2-24-10, Higashi-Toyoda**
**Hino-shi Tokyo(JP)**

(72) Erfinder: **Moriya, Koichi**
**39-15, Namiki-cho**
**Hachioji-shi Tokyo(JP)**

(72) Erfinder: **Hayakawa, Hidenori**
**3-3-19, Modori-cho**
**Musashino-shi Tokyo(JP)**

(74) Vertreter: **Schumacher, Günter, Dr. et al,**
**c/o Bayer AG Konzernverwaltung RP Patentabteilung**
**D-5090 Leverkusen 1 Bayerwerk(DE)**

(54) **Tetrahydrochonolin-1-ylcarbonylimidazol-Derivate, Zwischenprodukte für diese, Verfahren zur ihrer Herstellung und Herbizide oder Fungizide für Landwirtschaft und Gartenbau.**

(57) Die vorliegende Erfindung betrifft neue Tetrahydrochinolin-l-ylcarbonylimidazol-Derivate der Formel (I),

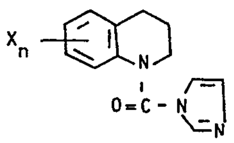

(I)

in welcher
X für Chlor steht und
n für 0, 1, 2 oder 3 steht,
Zwischenprodukte für diese, Verfahren zu ihrer Her-stellung und ihre Verwendung als Herbizide oder Fungizide für Landwirtschaft und Gartenbau.

**EP 0 158 954 A2**

NIHON TOKUSHU NOYAKU SEIZO K.K., Tokyo/Japan

# Tetrahydrochinolin-1-ylcarbonylimidazol-Derivate, Zwischenprodukte für diese, Verfahren zu ihrer Herstellung und Herbizide oder Fungizide für Landwirtschaft und Gartenbau

Die vorliegende Erfindung betrifft neue Tetrahydrochinolin-1-ylcarbonylimidazol-Derivate, Zwischenprodukte für diese, Verfahren zu ihrer Herstellung und Herbizide oder Fungizide für Landwirtschaft und Gartenbau.

Die vor dem Einreichungsdatum der vorliegenden Anmeldung bekannte US-PS 3 308 131 gibt eine Beschreibung über tertiäre Carbamyltriazole mit einer allgemeinen Formel, die die durch Formel (I) bezeichneten Verbindungen der vorliegenden Erfindung nicht einzuschließen vermag, und gibt an, daß jene Verbindungen insektizide Aktivität besitzen. Die US -PS offenbart speziell eine Verbindung der nachstehenden Formel.

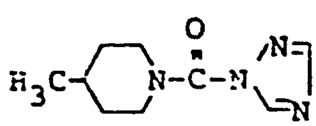

(A-1)

Die JP-PS 17748/1968 gibt eine Beschreibung über 2,4,5-Tribromoimidazol-Derivate mit einer allgemeinen Formel, die die durch Formel (I) bezeichneten Verbindungen der vorliegenden Erfindung nicht einzuschließen vermag, und

gibt an, daß jene Verbindungen insektizide und herbizide Aktivität besitzen. Die JP-PS offenbart speziell eine Verbindung der nachstehenden Formel.

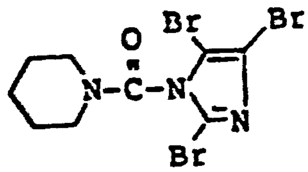

(B-1)

Seitens der Anmelderin wurden ausgedehnte Untersuchungen mit dem Ziel der Schaffung neuer, biologisch aktiver Verbindungen durchgeführt, die nunmehr zur erfolgreichen Synthese der Tetrahydrochinolin-1-ylcarbonylimidazol-Derivate geführt haben. Es wurde gefunden, daß diese Verbindungen biologische Aktivität, beispielsweise herbizide Aktivität und fungizide Aktivität, sowie Selektivität gegenüber Nutzpflanzen besitzen.

Es wurden nun neue Tetrahydrochinolin-1-ylcarbonyl-imidazol-Derivate der allgemeinen Formel (I),

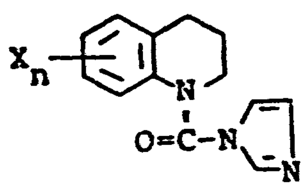

(I)

in der

X    ein Chlor-Atom bezeichnet und

n    für 0, 1, 2 oder 3 steht,

aufgefunden.

Die Verbindungen der allgemeinen Formel (I) können beispielsweise mittels der folgenden Verfahren hergestellt werden, auf die sich die vorliegende Erfindung ebenfalls erstreckt.

Verfahren i)

Ein Verfahren zur Herstellung der Verbindungen der allgemeinen Formel (I) umfaßt die Reaktion einer Verbindung der allgemeinen Formel

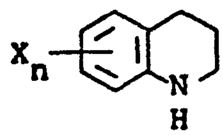

(II)

in der X und n die im Vorstehenden angegebenen Bedeutungen haben, mit einer Verbindung der nachstehenden Formel

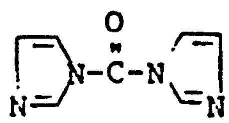

Verfahren ii)

Ein Verfahren zur Herstellung der Verbindungen der allgemeinen Formel (I) umfaßt die Reaktion einer Verbindung der allgemeinen Formel

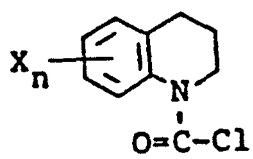

(III)

in der X und n die im Vorstehenden angegebenen Bedeutungen haben, mit Imidazol der nachstehenden Formel

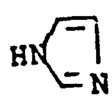

Die Untersuchungen der Anmelderin haben ergeben, daß die Verbindungen der allgemeinen Formel (I) gemäß der vorliegenden Erfindung neue Verbindungen sind, die in der Literatur vor der Einreichung der vorliegenden Anmeldung nicht beschrieben sind und in einfacher Weise hergestellt werden können, wie im Folgenden beschrieben wird. Weiterhin wurde gefunden, wie auch aus der nachstehenden Beschreibung hervorgeht, daß die Verbindungen der allgemeinen Formel (I) gemäß der vorliegenden Erfindung herbizide Aktivität besitzen, insbesondere eine ausgezeichnete Bekämpfungswirkung gegenüber Reisfeld-Unkräutern, und dabei gleichzeitig eine hervorragende Affinität zu Reispflanzen auf bewässerten Feldern.

Seitens der Anmelderin wurde weiterhin festgestellt, daß die erfindungsgemäßen Verbindungen Wirksamkeit bei der Bekämpfung von Pflanzenkrankheiten besitzen und beispielsweise eine ausgezeichnete Bekämpfungswirkung gegen die Reis-Braunfleckenkrankheit (Ophiobolus miyabeanus), den Kohl-Schwarzpilz (Alternaria brassicae), die Alternaria-Blattfleckenkrankheit des Apfels (Alternaria mali) und die Braunfäule verschiedener Pflanzen (Phytophthora infestans) zeigen.

Die vorliegende Erfindung erstreckt sich ebenfalls auf herbizide und fungizide Mittel für Landwirtschaft und Garbenbau, die die Verbindungen der allgemeinen Formel (I) als Wirkstoffe enthalten.

Gemäß der vorliegenden Erfindung wurde gefunden, daß die oben genannten, charakteristischen biologischen Eigenschaften der Verbindungen der Formel (I) gemäß der

vorliegenden Erfindung mit ihren chemischen charakteristischen Merkmalen zusammenhängen, das heißt mit ihrer spezifischen chemischen Struktur. Die Untersuchungen der Anmelderin haben ergeben, daß das charakteristische chemische Strukturmerkmal, wie aus der allgemeinen Formel (I) zu ersehen ist, darin liegt, daß das Stickstoff Atom in der 1-Stellung des 1,2,3,4-Tetrahydrochinolins, das durch ein oder mehrere Chlor-Atome substituiert sein kann, über eine Carbonyl-Gruppe mit einem Stickstoff-Atom des Imidazols verbunden ist. Es wurde gefunden, daß bei Substitution eines Chlor-Atoms in der 8-Stellung des Tetrahydrochinolins in der obigen Struktur eine ausgeprägte Korrelation zwischen der herbiziden Aktivität und der chemischen Struktur der erfindungsgemäßen Verbindungen besteht.

Die Erfindung betrifft weiterhin die Verbindungen der allgemeinen Formel (III), die bei der Herstellung der Verbindungen der allgemeinen Formel (I) verwendete Zwischenprodukte sind.

Die Verbindungen der allgemeinen Formel (III) können beispielsweise mittels des folgenden Verfahrens hergestellt werden, auf das sich die vorliegende Erfindung ebenfalls erstreckt.

<u>Verfahren iii)</u>

Ein Verfahren zur Herstellung der Verbindungen derallgemeinen Formel (III) umfaßt die Reaktion einer Verbindung der allgemeinen Formel (II) mit Phosgen oder Chlorameisensäure-trichloromethylester.

Die Verbindungen der allgemeinen Formel (III), die Zwischenprodukte für die Herstellung der Verbindungen der allgemeinen Formel (I) sind, sind ebenfalls neue Verbindungen, die in der vor der Einreichung der vorliegenden Anmeldung bekannten Literatur nicht beschrieben sind und als Zwischenprodukte für die durch die allgemeine Formel (I) bezeichneten Verbindungen gemäß der vorliegenden Erfindung wertvoll sind.

Ziel der vorliegenden Erfindung ist es, Tetrahydrochinolin-1-ylcarbonylimidazol-Derivate der allgemeinen Formel (I), Zwischenprodukte für diese, Verfahren zu ihrer Herstellung und die Verwendung dieser Verbindungen als Herbizide oder als Fungizide für Landwirtschaft und Gartenbau verfügbar zu machen.

Die vorgenannten und andere Ziele und Vorteile werden in der folgenden Beschreibung näher erläutert.

Da die durch die allgemeine Formel (I) bezeichneten Verbindungen gemäß der vorliegenden Erfindung durch eine niedrige Toxizität gegenüber warmblütigen Tieren und eine gute Selektivität gegenüber Kulturpflanzen, das heißt, Freiheit von Phytotoxizität gegenüber Kulturpflanzen bei den üblichen Dosierungen, gekennzeichnet sind, können sie beispielsweise vorteilhaft als Herbizide zur Unkrautbekämpfung verwendet werden. Die Herbizide gemäß der vorliegenden Erfindung zeigen insbesondere eine herausragende selektive Bekämpfungswirkung, wenn sie als Mittel zur Bodenbehandlung vor dem Auflaufen oder als Mittel zur Behandlung von Stengeln, Blättern und Boden gegen Reisfeld-Unkräuter eingesetzt werden.

Wie oben angegeben wurde, besitzen die Verbindungen der allgemeinen Formel (I) ausgezeichnete Sicherheit, hervorragende herbizide Aktivität und ein breites herbizides Spektrum.

Beispielsweise besitzen diese Verbindungen eine signifikante herbizide Aktivität gegenüber den folgenden Reisfeld-Unkräutern ohne irgendwelche schädigenden Wirkungen gegenüber den Reispflanzen.

| Pflanzenname: | Lateinische wissenschaftliche Bezeichnung: |
|---|---|
| Dikotyledonen | |
| Kikashigusa | Rotala indica Koehne |
| Falsche Pimpernelle (Büchsenkraut) | Lindernia procumbens Philcox |
| Falscher Weiderich (Heusenkraut) | Ludwigia prostrata Roxburgh |
| Breitblättriges Laichkraut | Potamogeton distinctus A. Bennett |
| Amerikanische Wasserwurz (Kreuztännel) | Elatine triandra Schk. |
| Monokotyledonen | |
| Hühnerhirse | Echinochloa crus-galli Beauv. var. oryzicola Ohwi |
| Monochoria | Monochoria vaginalis Presl. |

| | |
|---|---|
| Nadel-Sumpfbinse | Eleocharis acicularis L. |
| Wassernuß | Eleocharis kuroguwai Ohwi |
| Schirmkraut (Cyper-gras) | Cyperus difformis L. |
| Mizugayatsuri (Cypergras) | Cyperus serotinus Rottboell |
| Urikawa (Pfeilkraut) | Sagittaria pygmaea Miquel, |
| Schmalblättriger Wasserwegerich (Froschlöffel) | Alisma canaliculatum A. Braun et Bouché |
| Simse | Scirpus juncoides Roxburgh var. |

Weiterhin besitzen die Verbindungen der Formel (I) signifikante herbizide Aktivität gegen die folgenden, auf höher gelegenen Anbauflächen wachsenden Unkräuter.

| Pflanzenname: | Lateinische wissenschaftliche Bezeichnung: |
|---|---|
| **Dikotyledonen** | |
| Knöterich | Polygonum sp. |
| Gänsefuß | Chenopodium album Linnaeus |
| Vogelmiere | Stellaria media Villars |
| Kohl-Portulak | Portulaca oleracea Linnaeus |
| **Monokotyledonen** | |
| Hühnerhirse | Echinochloa crus-galli Beauv. var. |
| Fingergras | Digitaria adscendens Henr. |
| Chufa (Riedgras) | Cyperus iria L. |

Die im Vorstehenden angegebenen Pflanzen sind typische Beispiele für die jeweils mit der lateinischen wissenschaftlichen Bezeichnung genannte Gattung.

Die Einsetzbarkeit der durch die allgemeine Formel (I) bezeichneten Verbindungen gemäß der vorliegenden Erfindung ist nicht auf Unkräuter in bewässerten Reisfeldern und auf höher gelegenen landwirtschaftlichen Anbauflächen beschränkt, sondern sie sind auch wirksam gegen Unkräuter, die die Weichbinsen (Mattenbinsen) schädigen sowie Unkräuter, die auf zeitweise brach liegenden

Ländereien auftreten. Die hierin verwendete Bezeichnung "Unkräuter" bezeichnet im weitesten Sinne alle Pflanzen, die an Stellen auftreten, wo sie unerwünscht sind.

Die Verbindungen der allgemeinen Formel (I) gemäß der vorliegenden Erfindung zeigen ebenfalls starke fungizide Aktivität gegen einen breiten Bereich von Pilzen, die Pflanzenkrankheiten verursachen, sowie eine ausgezeichnete Rest-Wirkung und können dementsprechend auch zur Bekämpfung von Pflanzenkrankheiten eingesetzt werden.

Das fungizide Spektrum dieser Verbindungen zeigt, daß sie in wirksamer Weise zur Bekämpfung von Pflanzenkrankheiten verwendet werden können, die durch Archimycetes, Phycomycetes, Ascomycetes, Basidiomycetes und Fungi imperfecti sowie verschiedene Bakterien verursacht werden.

Im einzelnen sind die Verbindungen der vorliegenden Erfindung wertvoll zur Bekämpfung, beispielsweise, der Reis-Braunfleckenkrankheit (Ophiobolus miyabeanus), des Kohl-Schwarzpilzes (Alternaria brassicae), der Alternaria-Blattfleckenkrankheit des Apfels (Alternaria mali), der Schwarzfleckenkrankheit der Birne (Alternaria kikuchiana) der Braunfäule verschiedener Pflanzen (Phytophthora infestans) und der Brennfleckenkrankheit der Gurke und (einzulegenden) Orient-Melone (Colletotrichum lagenarium). Auch Reis-Bakterienbrand (Xanthomonas oryzae), der eine bedeutsame bakterielle Erkrankung darstellt, ist zu erwähnen.

Die Verbindungen der allgemeinen Formel (I) gemäß der vorliegenden Erfindung können in einfacher Weise zum

Beispiel mit Hilfe der folgenden Verfahren hergestellt werden.

Verfahren i)

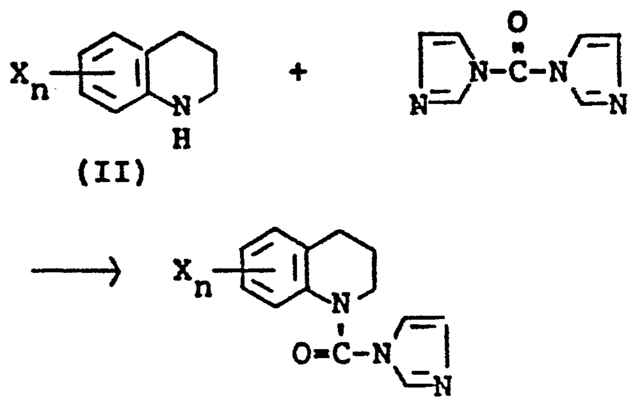

(II)

(I)

In dem obigen Schema haben X und n die im Vorstehenden angegebenen Bedeutungen.

Spezielle Beispiele für die als Ausgangsstoffe in dem durch das obige Reaktionsschema dargestellten Verfahren zur Herstellung der Verbindungen der allgemeinen Formel (I) eingesetzten Verbindungen der Formel (II) umfassen

1,2,3,4-Tetrahydrochinolin,
6-Chloro-1,2,3,4-tetrahydrochinolin,
8-Chloro-1,2,3,4-tetrahydrochinolin,
5,7-Dichloro-1,2,3,4-tetrahydrochinolin,
5,8-Dichloro-1,2,3,4-tetrahydrochinolin,
6,8-Dichloro-1,2,3,4-tetrahydrochinolin,
7,8-Dichloro-1,2,3,4-tetrahydrochinolin,
5,6-Dichloro-1,2,3,4-tetrahydrochinolin und
5,6,8-Trichloro-1,2,3,4-tetrahydrochinolin.

Das vorgenannte Verfahren wird durch das folgende typische Beispiel im einzelnen beschrieben.

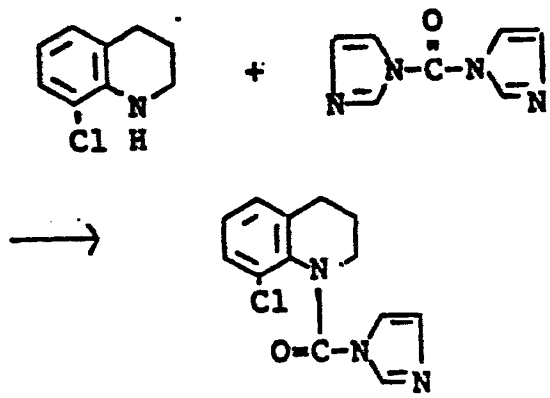

Zweckmäßigerweise kann das vorstehende Verfahren zur Herstellung der Verbindungen der allgemeinen Formel (I) gemäß der vorliegenden Erfindung unter Verwendung eines Lösungsmittels oder Verdünnungsmittels durchgeführt werden. Zu diesem Zweck können sämtliche inerten Lösungsmittel oder Verdünnungsmittel verwendet werden.

Beispiele für solche Lösungsmittel oder Verdünnungsmittel umfassen Wasser; aliphatische, alicyclische und aromatische Kohlenwasserstoffe (die gegebenenfalls chloriert sein können), wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol, Ether wie Diethylether, Methylethylether, Di-isopropylether, Dibutylether, Propylenoxid, Dioxan und Tetrahydrofuran, Ketone wie Aceton, Methylethylketon, Methylisopropylketon und Ethylisobutylketon, Nitrile wie Acetonitril, Propionitril und Acrylnitril, Alkohole wie Methanol, Ethanol, Isopropanol, Butanol und Ethylenglycol, Ester wie Ethylacetat und Amylacetat, Säureamide wie Dimethylformamid und Dimethylacetamid, Sulfone und Sulfoxide wie Dimethylsulfoxid und Sulfolan sowie Basen wie Pyridin.

Die vorstehende Reaktion kann in Gegenwart eines säurebindenden Mittels durchgeführt werden. Beispiele für das säurebindende Mittel umfassen die Hydroxide, Carbonate, Hydrogencarbonate und Alkoholate von Alkalimetallen sowie tertiäre Amine wie Triethylamin, Diethylanilin und Pyridin, die sämtlich allgemein verwendet werden.

Das obige Verfahren kann innerhalb eines breiten Temperaturbereichs durchgeführt werden. Im allgemeinen kann es bei einer Temperatur zwischen etwa -20°C und dem Siedepunkt der Mischung, zweckmäßigerweise bei einer Temperatur zwischen etwa 0°C und etwa 100°C, durchgeführt werden. Zweckmäßigerweise wird die Reaktion unter normalem Atmosphärendruck durchgeführt, jedoch ist es ebenfalls möglich, bei erhöhtem oder vermindertem Druck zu arbeiten.

<u>Verfahren ii)</u>

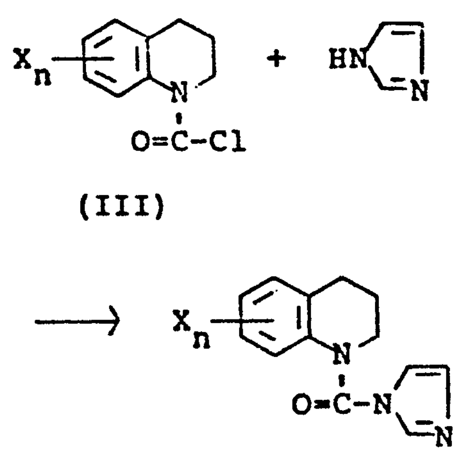

(III)

(I)

In dem obigen Schema haben X und n die im Vorstehenden angegebenen Bedeutungen.

Spezielle Beispiele für die als Ausgangsstoffe in dem durch das obige Reaktionsschema dargestellten Verfahren zur Herstellung der Verbindungen der allgemeinen Formel (I) gemäß der vorliegenden Erfindung eingesetzten Verbindungen der Formel (III) umfassen

1(H),2,3,4-Tetrahydrochinolin-1-ylcarbonylchlorid,
6-Chloro-1(H),2,3,4-tetrahydrochinolin-1-ylcarbonylchlorid,
8-Chloro-1(H),2,3,4-tetrahydrochinolin-1-ylcarbonylchlorid,
5,7-Dichloro-1(H),2,3,4-tetrahydrochinolin-1-ylcarbonylchlorid,
5,8-Dichloro-1(H),2,3,4-tetrahydrochinolin-1-ylcarbonylchlorid,
6,8-Dichloro-1(H),2,3,4-tetrahydrochinolin-1-ylcarbonylchlorid,
7,8-Dichloro-1(H),2,3,4-tetrahydrochinolin-1-ylcarbonylchlorid,
5,6-Dichloro-1(H),2,3,4-tetrahydrochinolin-1-ylcarbonylchlorid und
5,6,8-Trichloro-1(H),2,3,4-tetrahydrochinolin-1-ylcarbonylchlorid.

Das vorgenannte Verfahren wird durch das folgende typische Beispiel im einzelnen beschrieben.

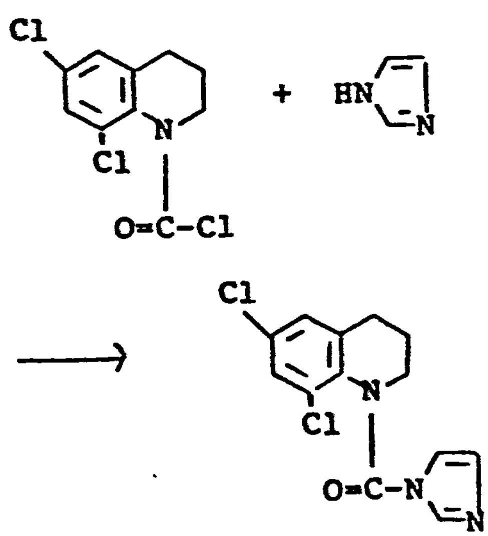

Nit 180

Zur Durchführung des obigen Verfahrens werden zweckmäßigerweise die gleichen Lösungsmittel oder Verdünnungsmittel verwendet, wie sie im Vorstehenden beispielhaft angegeben sind, und das gewünschte Produkt hoher Reinheit kann in hoher Ausbeute erhalten werden.

Das obige Verfahren kann innerhalb eines breiten Temperaturbereichs durchgeführt werden, im allgemeinen bei einer Temperatur zwischen -20°C und dem Siedepunkt der Mischung, vorzugsweise bei einer Temperatur zwischen 0°C und 100°C. Zweckmäßigerweise wird die Reaktion unter normalem Atmosphärendruck durchgeführt, jedoch ist es ebenfalls möglich, bei erhöhtem oder vermindertem Druck zu arbeiten.

Die Verbindungen der allgemeinen Formel (III) gemäß der vorliegenden Erfindung als Zwischenprodukte für die Herstellung der Verbindungen der allgemeinen Formel (I) können zum Beispiel mit Hilfe des folgenden Verfahrens iii) hergestellt werden.

Verfahren iii)

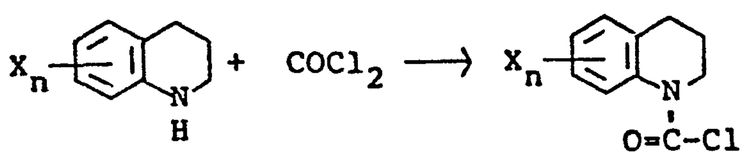

(II)

(III)

In dem obigen Schema haben X und n die im Vorstehenden angegebenen Bedeutungen.

Spezielle Beispiele für die als Ausgangsstoffe in dem durch das obige Reaktionsschema dargestellten Verfahren

zur Herstellung der Verbindungen der allgemeinen Formel (III) gemäß der vorliegenden Erfindung eingesetzten Verbindungen der Formel (II) können die gleichen sein, wie sie oben für das Verfahren i) beispielhaft genannt sind.

Als anderer Ausgangsstoff kann Chlorameisensäure-trichloromethylester ($ClCOOCCl_3$) an Stelle von Phosgen umgesetzt werden.

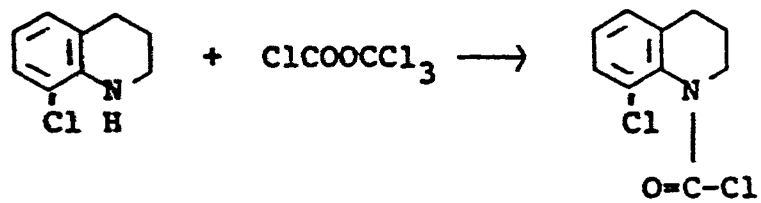

Zur Durchführung des obigen Verfahrens werden zweckmäßigerweise die gleichen Lösungsmittel oder Verdünnungsmittel verwendet, wie sie im Vorstehenden beispielhaft angegeben sind, und das gewünschte Produkt hoher Reinheit kann in hoher Ausbeute erhalten werden.

Das obige Verfahren kann innerhalb eines breiten Temperaturbereichs durchgeführt werden, im allgemeinen bei einer Temperatur zwischen -20°C und dem Siedepunkt der Mischung, vorzugsweise bei einer Temperatur zwischen 0°C und 100°C. Zweckmäßigerweise wird die Reaktion unter normalem Atmosphärendruck durchgeführt, jedoch ist es ebenfalls möglich, bei erhöhtem oder vermindertem Druck zu arbeiten.

Als Herbizide oder Fungizide für Landwirtschaft und Gartenbau können die Verbindungen gemäß der vorliegenden Erfindung unmittelbar nach dem Verdünnen mit Wasser

oder aber in Form verschiedenartiger Formulierungen zur Anwendung gebracht werden, wie sie unter Verwendung landwirtschaftlich unbedenklicher Hilfsstoffe durch Verfahren gewonnen werden, die bei der praktischen Herstellung von Agrochemikalien allgemein angewandt werden. Im praktischen Gebrauch werden diese verschiedenen Formulierungen entweder unmittelbar oder nach dem Verdünnen mit Wasser auf die gewünschte Konzentration eingesetzt.

Zu den hierin erwähnten landwirtschaftlich unbedenklichen Hilfsstoffen zählen beispielsweise Verdünnungsmittel (Lösungsmittel, Streckmittel, Träger), oberflächenaktive Mittel (Lösungsvermittler, Emulgatoren, Dispergiermittel, Netzmittel), Stabilisatoren, Haftmittel, Aerosol-Treibmittel und synergistische Mittel.

Beispiele für die Lösungsmittel sind Wasser und organische Lösungsmittel, beispielsweise Kohlenwasserstoffe [z.B. n-Hexan, Petrolether, Erdöl-Fraktionen (z.B. Paraffinwachse, Kerosin, Leichtöle, Mittelöle und Schweröle), Benzol, Toluol und Xylol], halogenierte Kohlenwasserstoffe (z.B. Methylenchlorid, Kohlenstofftetrachlorid, Ethylenchlorid, Ethylendibromid, Chlorbenzol und Chloroform), Alkohole (z.B. Methanol, Ethanol, Propanol und Ethylenglycol), Ether (z.B. Diethylether, Ethylenoxid und Dioxan), Alkohol-ether (z.B. Ethylenglycol-monomethylether), Ketone (z.B. Aceton und Isophoron), Ester (z.B. Ethylacetat und Amylacetat), Amide (z.B. Dimethylformamid und Dimethylacetamid) und Sulfoxide (z.B. Dimethylsulfoxid).

Beispiele für die Streckmittel oder Träger umfassen anorganische Pulver, beispielsweise Löschkalk, Magnesiumkalk, Gips, Calciumcarbonat, Siliciumdioxid, Perlit, Bimsstein, Calcit, Diatomeenerde, amorphes Siliciumdioxid, Aluminiumoxid, Zeolithe und Tonminerale (z.B. Pyrophyllit, Talkum, Montmorillonit, Beidellit, Vermikulit, Kaolinit und Glimmer), pflanzliche Pulver wie Getreidemehle, Stärkearten, verarbeitete Stärkearten, Zucker, Glucose und zerkleinerte Stengel von Pflanzen, sowie Pulver aus synthetischen Harzen wie Phenol-Harzen, Harnstoff-Harzen und Vinylchlorid-Harzen.

Beispiele für die oberflächenaktiven Mittel umfassen anionische oberflächenaktive Mittel wie Alkylsulfonsäureester (z.B. Natriumlaurylsulfat), Arylsulfonsäuren (z.B. Alkylarylsulfonsäure-Salze und Natriumalkylnaphthalinsulfonate), Bernsteinsäure-Salze und Salze von Schwefelsäureestern von Polyethylenglycol-alkylarylethern, kationische oberflächenaktive Mittel wie Alkylamine (z.B. Laurylamin, Stearyltrimethylammoniumchlorid und Alkyldimethylbenzylammoniumchloride) und Polyoxyethylenalkylamine, nicht-ionische oberflächenaktive Mittel wie Polyoxyethylenglycolether (z.B. Polyoxyethylenalkylarylether und deren Kondensationsprodukte), Polyoxyethylenglycolester (z.B. Polyoxyethylenfettsäureester) und Ester mehrwertiger Alkohole (z.B. Polyoxyethylensorbitan-monolaurat) sowie amphotere oberflächenaktive Mittel.

Beispiele für andere Hilfsstoffe umfassen Stabilisatoren, Haftmittel (z.B. landwirtschaftliche Seifen, Casein-Kalk, Natriumalginat, Polyvinylalkohol, Haftmittel vom Vinylacetat-Typ und Acryl-Haftmittel),

Aerosol-Treibmittel (z.B. Trichlorofluoromethan, Dichlorofluoromethan, 1,2,2-Trichloro-1,1,2-trifluoroethan, Chlorbenzol, verflüssigtes Erdgas (LNG) und niedere Ether), die Verbrennung steuernde Mittel für Räuchermittel (z.B. Nitrite, Zink-Pulver und Dicyandiamid), sauerstoff-abgebende Mittel (z.B. Chlorate), wirkungsverlängernde Mittel, Dispersions-Stabilisatoren /¯z.B. Casein, Tragant, Carboxymethylcellulose (CMC) und Polyvinylalkohol (PVA)_7 und synergistische Mittel.

Die Verbindungen der vorliegenden Erfindung können mittels der allgemein auf dem Gebiet der Agrochemikalien gebräuchlichen Verfahren zu verschiedenen Präparaten formuliert werden. Erläuternde Beispiele für solche Anwendungsformen sind emulgierbare Konzentrate, Öl-Präparate, benetzbare Pulver, lösliche Pulver, Suspensionen, Stäubemittel, Granulate, pulvrige Präparate, Räuchermittel, Tabletten, Aerosole, Pasten und Kapseln.

Die Herbizide oder Fungizide für Landwirtschaft und Gartenbau gemäß der vorliegenden Erfindung können etwa 0,001 bis etwa 100 Gew.-%, vorzugsweise etwa 0,005 bis etwa 95 Gew.-% des vorerwähnten Wirkstoffes enthalten.

Für den praktischen Gebrauch beträgt die geeignete Menge der aktiven Verbindung in den vorgenannten verschiedenartigen Formulierungen und gebrauchsfertigen Präparaten im allgemeinen etwa 0,01 bis etwa 95 Gew.-%, vorzugsweise etwa 0,05 bis etwa 60 Gew.-%.

Der Gehalt des Wirkstoffs kann in geeigneter Weise je nach der Art der Formulierung, dem Verfahren, Zweck, der Zeit und dem Ort seiner Anwendung sowie dem Zustand

des Auftretens der schädlichen Insekten variiert werden.

Erforderlichenfalls können die Verbindungen gemäß der vorliegenden Erfindung auch in Kombination mit anderen Agrochemikalien verwendet werden, beispielsweise mit Insektiziden, anderen Fungiziden, Mitiziden, Nematiziden, Anti-Virus-Mitteln, anderen Herbiziden, Pflanzen-Wachstumsregulatoren und Lockstoffen /¯z.B. Organophosphat-Verbindungen, Carbamat-Verbindungen, Dithio(oder thiol)carbamat-Verbindungen, Organochlor-Verbindungen, Dinitro-Verbindungen, organischen Schwefel- oder metallorganischen Verbindungen, Antibiotika, substituierten Diphenylether-Verbindungen, Harnstoff-Verbindungen und Triazin-Verbindungen_7 und/ oder Düngemitteln.

Den im Vorstehenden bezeichneten erfindungsgemäßen Wirkstoff enthaltende verschiedenartige Mittel und gebrauchsfertige Präparate können mittels verschiedener Verfahren zur Anwendung gebracht werden, wie sie allgemein für das Aufbringen von Agrochemikalien gebräuchlich sind, beispielsweise durch Sprühen (Versprühen von Flüssigkeiten, Vernebeln, Zerstäuben, Stäuben, Streuen von Granulat, Wasser-Oberflächenbehandlung, Gießen etc.), Räuchern, Bodenbehandlung (Vermischen mit dem Boden, Spritzen, Bedampfen, Gießen etc.), Oberflächen-Anwendung (z.B. Beschichten, Aufbringen in Form von Bändern, Pulverbeschichten, Bedecken etc.) und Eintauchen. Sie können auch mittels des sogenannten Ultra-Low-Volume-Sprühverfahrens aufgebracht werden. Nach diesem Verfahren kann der Wirkstoff sogar in einer Konzentration von 100 % eingearbeitet sein.

Die Aufwandmenge pro Flächeneinheit beträgt beispielsweise etwa 0,5 bis etwa 8 kg/ha, vorzugsweise etwa 1 bis etwa 5 kg/ha für das Herbizid und etwa 1 bis etwa 10 kg/ha für das Fungizid für Landwirtschaft und Gartenbau. In besonders gelagerten Fällen können oder sollten sogar die Aufwandmengen außerhalb des angegebenen Bereichs liegen.

Gemäß der vorliegenden Erfindung kann ein herbizides Mittel oder ein fungizides Mittel für Landwirtschaft und Gartenbau zur Verfügung gestellt werden, das als Wirkstoff eine Verbindung der allgemeinen Formel (I) sowie ein Verdünnungsmittel (ein Lösungsmittel und/oder ein Streckmittel und/oder einen Träger) und/oder ein oberflächenaktives Mittel und, falls weiterhin erforderlich, einen Stabilisator, ein Haftmittel, ein synergistisches Mittel etc. enthält.

Die vorliegende Erfindung macht weiterhin ein Verfahren zur Bekämpfung von Unkräutern und Pflanzenkrankheiten verfügbar, das darin besteht, daß eine Verbindung der allgemeinen Formel (I) allein oder in Form einer Mischung mit einem Verdünnungsmittel (einem Lösungsmittel und/oder einem Streckmittel und/oder einem Träger) und/oder einem oberflächenaktiven Mittel und, falls weiterhin erforderlich, einem Stabilisator, einem Haftmittel, einem synergistischen Mittel etc. auf Unkräuter, Pflanzen-Pathogene und/oder den Ort ihres Auftretens aufgebracht wird.

Die vorliegende Erfindung wird durch die folgenden Beispiele im einzelnen erläutert, ist jedoch nicht auf diese speziellen Beispiele allein beschränkt.

Beispiel 1

8-Chloro-1,2,3,4-tetrahydrochinolin (2,3 g) und N,N'-Carbonyldiimidazol (1,8 g) wurden unter Rühren 24 h in Toluol (20 ml) erhitzt. Das Lösungsmittel wurde unter vermindertem Druck abgedampft, und der Rückstand wurde bei 80°C und 1,33 mbar (1 mmHg) konzentriert. Dem Rückstand wurde Wasser zugesetzt, um ihn zur Kristallisation zu bringen. Die Suspension wurde filtriert, wonach das gewünschte 8-Chloro-1(H),2,3,4-tetrahydrochinolin-1-ylcarbonylimidazol (2,2 g) der nachstehenden Formel in Form von Kristallen erhalten wurde. Das Produkt wurde aus Ether umkristallisiert; Schmp. 122-123°C.

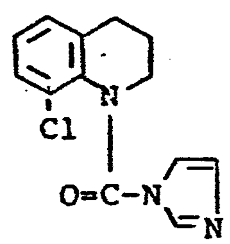

(Verbindung Nr. 1)

Beispiel 2

6,8-Dichloro-1(H),2,3,4-tetrahydrochinolin-1-ylcarbonylchlorid (2,63 g) und Imidazol (2,0 g) wurden in Tetrahydrofuran (20 ml) 16 h unter Rückfluß-Temperatur gerührt. Tetrahydrofuran und überschüssiges Imidazol wurden unter vermindertem Druck abgedampft. Dem Rückstand wurde Wasser zugesetzt, um ihn zur Kristallisation zu bringen. Die Suspension wurde filtriert, wonach das gewünschte 6,8-Dichloro-1(H),2,3,4-tetrahydrochinolin-1-ylcarbonylimidazol (2,5 g) der nachstehenden

Formel in Form von Kristallen erhalten wurde. Das Produkt wurde aus Ether umkristallisiert; Schmp. 137-138°C.

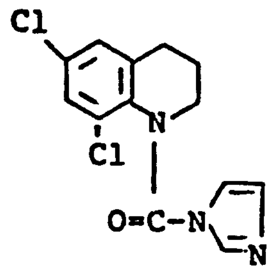

(Verbindung Nr. 2)

Die nachstehende Tabelle 1 zeigt Verbindungen der allgemeinen Formel (I) gemäß der vorliegenden Erfindung, die mittels der gleichen Verfahren wie in den Beispielen 1 oder 2 synthetisiert wurden.

Tabelle 1

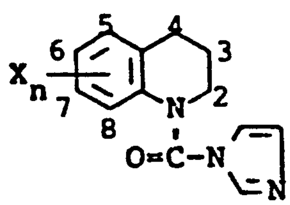

| Verbindung Nr. | $X_n$ | Physikalische Konstante |
|---|---|---|
| 3 | - | Schmp. 97-98°C |
| 4 | 6-Cl | Schmp. 125-127°C |
| 5 | 5,7-$Cl_2$ | Schmp. 123-124°C |
| 6 | 5,8-$Cl_2$ | Schmp. 177-178°C |
| 7 | 7,8-$Cl_2$ | Schmp. 130-132°C |
| 8 | 5,6,8-$Cl_3$ | Schmp. 150-152°C |

Anmerkung:
In der Tabelle bezeichnet "-" für die Verbindung Nr. 3 in der Spalte $X_n$, daß diese keinen Substituenten hat.

Beispiel 3

Synthese des Zwischenprodukts:

Chlorameisensäure-trichloromethylester (20 ml) wurde in Ethylacetat (100 ml) gelöst, und die Lösung wurde auf 50°C bis 60°C (Ölbad-Temperatur) erwärmt. Unter Rühren wurde eine Lösung von 6,8-Dichloro-1,2,3,4-tetrahydrochinolin (10 g) in Ethylacetat (20 ml) im Laufe von 30 min hinzugefügt. Die Reaktions-Temperatur wurde dann allmählich bis auf die Rückfluß-Temperatur erhöht, und die Mischung wurde 5 h gerührt. Das Lösungsmittel wurde auf dem Dampfbad abgedampft, und der Rückstand wurde unter vermindertem Druck konzentriert, wonach das gewünschte 6,8-Dichloro-1(H),2,3,4-tetrahydrochinolin-1-ylcarbonylchlorid (10,5 g) der nachstehenden Formel erhalten wurde. Das Produkt wurde aus Ether umkristallisiert; Schmp. 67-68°C.

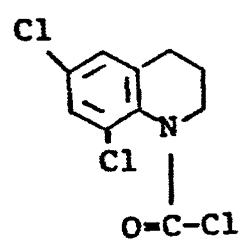

(Verbindung Nr. III-2)

Die Tabelle 2 zeigt Verbindungen der allgemeinen Formel (III), die mittels des gleichen Verfahrens wie in Beispiel 3 synthetisiert wurden.

## Tabelle 2

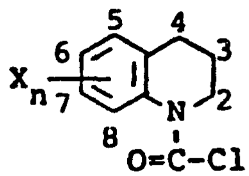

| Verbindung | $X_n$ | Physikalische Konstante |
|---|---|---|
| III-1 | 8-Cl | Sdp. 143-145°C/0,67 mbar (0,5 mmHg) |
| III-3 | - | Sdp. 136-140°C/0,67 mbar (0,5 mmHg) |
| III-4 | 6-Cl | Sdp. 146-148°C/0,67 mbar (0,5 mmHg) |
| III-5 | 5,7-$Cl_2$ | Sdp. 168-170°C/0,67 mbar (0,5 mmHg) |
| III-6 | 5,8-$Cl_2$ | Schmp. 98-100°C |
| III-7 | 7,8-$Cl_2$ | Sdp. 165-167°C/0,67 mbar (0,5 mmHg) |
| III-8 | 5,6,8-$Cl_3$ | Sdp. 178-180°C/0,67 mbar (0,5 mmHg) |

Anmerkung:

In der Tabelle hat "-" in der Spalte $X_n$ die gleiche Bedeutung wie in Tabelle 1.

## Beispiel 4 (Benetzbares Pulver)

15 Teile der Verbindung Nr. 1 der Erfindung, 80 Teile eines 1:5-Gemisches aus pulvriger Diatomeenerde und Tonpulver, 2 Teile Natriumalkylbenzolsulfonat und 3 Teile Natriumalkylnaphthalinsulfonat/Formaldehyd-Kondensat werden pulverisiert und zu einem benetzbaren Pulver vermischt. Dieses wird mit Wasser verdünnt und auf Unkräuter, Pathogene und/oder den Ort ihres Auftretens aufgesprüht.

Beispiel 5 (Emulgierbares Konzentrat)

30 Teile der Verbindung Nr. 2 der Erfindung, 55 Teile Xylol, 8 Teile Polyoxyethylen-alkylphenylether und 7 Teile Calciumalkylbenzolsulfonat werden unter Rühren miteinander vermischt, wodurch ein emulgierbares Konzentrat hergestellt wird. Dieses wird mit Wasser verdünnt und auf Unkräuter, Pathogene und/oder den Ort ihres Auftretens aufgesprüht.

Beispiel 6 (Stäubemittel)

2 Teile der Verbindung Nr. 3 der Erfindung und 98 Teile Tonpulver werden pulverisiert und gemischt, wodurch ein Stäubemittel hergestellt wird. Dieses wird über Unkräutern, Pathogenen und/oder dem Ort ihres Auftretens ausgestreut.

Beispiel 7 (Stäubemittel)

Die Verbindung Nr. 2 der Erfindung (1,5 Teile), 0,5 Teile Isopropyl-hydrogenphosphat (PAP) und 98 Teile Tonpulver werden pulverisiert und gemischt, wodurch ein Stäubemittel hergestellt wird. Dieses wird über Unkräutern, Pathogenen und/oder dem Ort ihres Auftretens ausgestreut.

Beispiel 8 (Granulat)

Wasser (25 Teile) wird zu einer Mischung aus 10 Teilen der Verbindung Nr. 1 der Erfindung, 30 Teilen Bentonit (Montmorillonit), 58 Teilen Talkum und 2 Teilen Ligninsulfonat zugesetzt, und das Gemisch wird gut geknetet.

Die Mischung wird mittels eines Extruder-Granulators zu einem Granulat mit einer Korngröße von 0,43 bis 2,0 mm (10 bis 40 mesh) verarbeitet, das dann bei 40°C bis 50°C getrocknet wird, wodurch ein Granulat gebildet wird. Das Granulat wird über Unkräutern, Pathogenen und/oder dem Ort ihres Auftretens ausgestreut.

Beispiel 9 (Granulat)

Ein Drehmischer wird mit 95 Teilen Tonmineral-Teilchen mit einer Teilchengrößen-Verteilung zwischen 0,2 und 2 mm beschickt, und unter Drehen des Mischers werden 5 Teile der Verbindung Nr. 2 der Erfindung auf die Teilchen zur gleichmäßigen Benetzung derselben aufgesprüht, wodurch ein Granulat gebildet wird. Das Granulat wird über Unkräutern, Pathogenen und/oder dem Ort ihres Auftretens ausgestreut.

Beispiel 10 (Biologischer Test)

Prüfung der Wirkung gegen im Wasser wachsende Reisfeld-Unkräuter durch Behandlung des Bodens sowie der Stengel und Blätter unter Bewässerungs-Bedingungen (Topf-Test):

---

Herstellung eines Wirkstoff-Präparats:

Träger: 5 Gewichtsteile Aceton;
Emulgator: 1 Gewichtsteil Benzyloxypolyglycolether.

Ein die aktive Verbindung enthaltendes Präparat wird durch Mischen von 1 Gew.-Teil jeder der aktiven Verbindungen mit dem Träger und dem Emulgator in den vorbezeichneten Mengen und Verdünnen einer vorher festgelegten Menge des emulgierbaren Konzentrats mit Wasser hergestellt.

Test-Verfahren

Reis-Kulturboden wurde in Wagner-Töpfe (2 $dm^2$) gefüllt, und zwei Reis-Setzlinge (Varietät: Kinnampu) im 2- bis 3-Blattstadium (Pflanzenhöhe etwa 10 cm) wurden in jeden Topf umgepflanzt. Samen von Echinochloa crus-galli, Cyperus microiria, Monochoria vaginalis, Scirpus juncoides und breitblättrigen Unkräutern, kleine Stücke von Eleocharis acicularis und Knollen von Cyperus serotinus und Sagittaria pygmaea wurden eingesetzt, und die Töpfe wurden in feuchtem Zustand gehalten. Als Echinochloa crus-galli etwa bis zum 2-Blatt-Stadium gewachsen war (etwa 7 bis 9 Tage nach der Einsaat), wurden die Töpfe mit Wasser bis zu einer Tiefe von etwa 6 cm gefüllt. Eine vorher festgelegte Menge der erfindungsgemäßen Verbindung wurde in Form einer Emulsion mittels einer Pipette zur Behandlung jedes Topfes aufgebracht. Nach der Behandlung wurde das Wasser im Laufe von zwei Tagen mit einer Geschwindigkeit von 2 bis 3 cm pro Tag aus den Töpfen heraussickern gelassen. Danach wurde die Wassertiefe in den Töpfen bei etwa 3 cm gehalten. In der vierten Wochen nach der Behandlung mit der Chemikalie wurden die herbizide Wirkung und der Grad der Phytotoxizität mit Hilfe einer Skala von 0 bis 5 nach dem folgenden Maßstab bewertet.

Bewertung der herbiziden Wirkung: Unkrautvernichtungsrate (bezogen auf die unbehandelten Flächen)

| | |
|---|---|
| 5 | wenigstens 95 % (verdorrt) |
| 4 | wenigstens 80 %, jedoch weniger als 95 % |
| 3 | wenigstens 50 %, jedoch weniger als 80 % |
| 2 | wenigstens 30 %, jedoch weniger als 50 % |
| 1 | wenigstens 10 %, jedoch weniger als 30 % |
| 0 | weniger als 10 % (unwirksam) |

Bewertung der Phytotoxizität gegenüber im Wasser befindlichen Reispflanzen: Phytotoxizitätsrate (bezogen auf die unbehandelten Flächen)

| | |
|---|---|
| 5 | wenigstens 90 % (Ausrottung) |
| 4 | wenigstens 50 %, jedoch weniger als 90 % |
| 3 | wenigstens 30 %, jedoch weniger als 50 % |
| 2 | wenigstens 10 %, jedoch weniger als 30 % |
| 1 | mehr als 0 %, jedoch weniger als 10 % |
| 0 | 0 % (keine Phytotoxizität) |

Die Testergebnisse sind in Tabelle 3 aufgeführt.
Die Symbole A bis H bezeichnen die nachstehenden Unkräuter:

A: Echinochloa crus-galli
B: Eleocharis acicularis
C: Cyperus microiria
D: Scirpus juncoides
E: Monochoria vaginalis
F: Breitblättrige Unkräuter (Lindernia procumbens, Rotala indica, Elatine triandra etc.)
G: Cyperus serotinus
H: Sagittaria pygmaea.

Tabelle 3

| Verbindung | Wirkstoff-Menge kg/ha | Herbizide Wirkung Unkräuter A | B | C | D | E | F | G | H | Phytotoxität gegen Reis |
|---|---|---|---|---|---|---|---|---|---|---|
| 1 | 20 | 5 | 5 | 5 | 5 | 5 | 5 | 5 | 5 | 0 |
| 2 | 20 | 5 | 5 | 5 | 5 | 5 | 5 | 5 | 5 | 0 |
| 6 | 20 | 5 | 5 | 5 | 5 | 5 | 5 | 5 | 5 | 0 |
| 7 | 20 | 5 | 5 | 5 | 5 | 5 | 5 | 5 | 5 | 0 |
| 8 | 20 | 5 | 5 | 5 | 5 | 5 | 5 | 5 | 5 | 0 |
| Kontrolle | | | | | | | | | | |
| A-1 | 20 | 0 | 0 | 1 | 0 | 0 | 1 | 0 | 0 | 0 |
| B-1 | 20 | 0 | 0 | 1 | 0 | 1 | 2 | 0 | 0 | 0 |

Anmerkung:

Kontrolle A-1: Die in der oben erwähnten US-PS 3 308 131 beschriebene Verbindung.

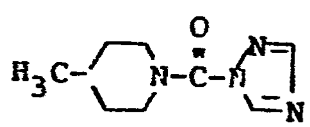

Kontrolle B-1: Die in der oben erwähnten JP-PS 17748/1968 beschriebene Verbindung.

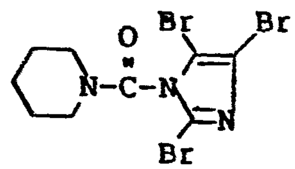

Nit 180

Beispiel 11 (Biologischer Test)

Prüfung der Wirkung gegen Cochliobolus miyabeanus durch Besprühen der Stengel und Blätter:

---

Reis-Pflanzen (Varietät: Kusabue) wurden in unglasierten Töpfen mit einem Durchmesser von 12 cm kultiviert, und im 3- bis 4-Blatt-Stadium wurde eine gemäß Beispiel 10 hergestellte Verdünnung der Test-Verbindung mit einer Rate von 50 ml auf jeweils drei Töpfe gesprüht. Am folgenden Tag wurde eine Suspension künstlich kultivierter Sporen von Cochliobolus miyabeanus zweimal auf die Töpfe gesprüht. Die Töpfe wurden zur Herbeiführung der Infektion in einer feuchten Kammer mit einer relativen Luftfeuchtigkeit von 100 % und einer Temperatur von 25°C gehalten. Sieben Tage nach der Inokulierung wurde der Grad der Erkrankung pro Topf mit Hilfe eines Maßstabs von 0 bis 5 wie folgt bewertet, und der Bekämpfungs-Index (%) wurde berechnet.

| Grad der Erkrankung | Ausmaß der Erkrankung |
|---|---|
| 0 | keine Erkrankung |
| 1 | geringfügig |
| 2 | klein |
| 3 | mittel |
| 4 | groß |
| 5 | sehr groß |

Nit 180

$$\text{Bekämpfungs-Index (\%)} = \frac{\text{Grad der Erkrankung einer unbehandelten Fläche} - \text{Grad der Erkrankung einer behandelten Fläche}}{\text{Grad der Erkrankung einer unbehandelten Fläche}} \times 100$$

In diesem Test bildeten jeweils drei Töpfe eine Partie. Die Verbindungen Nr. 1, 2 und 4 zeigten bei einer Konzentration der aktiven Komponente von 500 ppm einen Bekämpfungs-Index von 100 %. Die Kontroll-Verbindungen A-1 und B-1 aus Beispiel 10 zeigten einen Bekämpfungs-Wert von weniger als 10 % bei einer Konzentration der aktiven Komponente von 500 ppm.

Beispiel 12 (Biologischer Test)

Test auf Bekämpfung der Braunfäule der Tomate (Phytophthora infestans):

---

Jede der Test-Verbindungen in Form einer Emulsion, die gemäß Beispiel 10 hergestellt worden war, wurde auf in unglasierten 9 cm-Töpfen gezogene Tomaten (Varietät: "Kurihara") mit einer Sprühpistole aufgesprüht. Einen Tag nach dem Sprühen wurden die Pflanzen mit einer Sporen-Suspension des oben genannten Pathogens durch Sprühen inokuliert, und die Pflanzen wurden über Nacht in einem Raum mit einer konstanten Temperatur von 22°C und einer Luftfeuchtigkeit von wenigstens 90 % stehen gelassen. Fünf Tage später wurde der Grad der Erkrankung durch das Verhältnis der Läsionen zeigenden Flächen bestimmt, und der Bekämpfungsindex wurde berechnet.

| Grad der Erkrankung | Fläche der Verletzungen (%) |
|---|---|
| 0 | 0 |
| 0,5 | 2 oder weniger |
| 1 | 3 bis 5 |
| 2 | 6 bis 15 |
| 3 | 16 bis 30 |
| 4 | 31 bis 50 |
| 5 | 51 oder mehr |

$$\text{Bekämpfungs-Index (\%)} = \frac{\text{Grad der Erkrankung einer unbehandelten Fläche} - \text{Grad der Erkrankung einer behandelten Fläche}}{\text{Grad der Erkrankung einer unbehandelten Fläche}} \times 100$$

Die Verbindung Nr. 3 zeigte bei einer Konzentration der aktiven Komponente von 500 ppm eine Bekämpfungswirkung von 100 %. Die Kontroll-Verbindungen A-1 und B-1 zeigten einen Bekämpfungs-Wert von 0 %.

Beispiel 13 (Biologischer Test)

Test auf Bekämpfung der Alternaria brassicae auf Gemüse der Cruciferae-Arten:

Eine wie in Beispiel 10 hergestellte Chemikalie mit vorher festgelegter Konzentration wurde in einer Rate von 25 ml pro drei Töpfe auf kleine Sämlinge von Chinakohl aufgesprüht, die nach der Einsaat in 9 cm-Töpfen aus Vinyl-Kunststoff gezogen worden waren. An dem auf

0158954

das Sprühen folgenden Tage wurde eine Suspension von Conidiosporen des oben bezeichneten Pathogens, die durch Kultivieren in einem Kartoffel-Agar-Medium erhalten worden war, durch Sprühen auf den behandelten Chinakohl inokuliert, und die Töpfe wurden einen Tag in einer feuchten Kammer (relative Luftfeuchtigkeit: 100 %) bei 25°C gehalten. Danach wurden sie zur Infizierung in ein Glas-Gewächshaus (20°C bis 30°C) überführt. Sieben Tage nach dem Inokulieren wurde der Grad der Erkrankung nach dem gleichen Maßstab wie in Beispiel 10 bewertet, und der Bekämpfungs-Index wurde berechnet. Es wurde gefunden, daß beispielsweise die Verbindung Nr. 4 bei einer Konzentration der aktiven Komponente von 500 ppm einen Bekämpfungs-Index von 100 % zeigte, während die Kontroll-Verbindungen A-1 und B-1 bei 500 ppm einen Bekämpfungs-Wert von 0 % zeigten.

Beispiel 14 (Biologischer Test)

Test auf Bekämpfung der Alternaria mali auf Äpfeln:

Eine Lösung mit vorher festgelegter Konzentration jeder der Test-Verbindungen wurde mit einer Rate von 100 ml pro drei Töpfe auf junge Apfel-Sämlinge (Varietät: Star King; zweijährige Sämlinge) aufgesprüht, die in unglasierten Töpfen #3 gezogen worden waren und etwa 10 neu entwickelte Blätter hatten.

An dem auf das Sprühen folgenden Tage wurde eine Suspension von Conidiosporen von Alternaria mali (die 7 Tage in einem Aprikosen-Medium kultiviert worden waren) auf die behandelten Apfel-Sämlinge durch Sprühen inokuliert, und die Töpfe wurden zwei Tage in einer feuchten Kammer bei 25°C gehalten.

Drei Tage nach dem Inokulieren wurde die Anzahl Läsionen für zehn Blätter in jedem der Töpfe untersucht, und die mittlere Zahl der Läsionen pro Blatt wurde berechnet. Der Bekämpfungsindex wurde nach der folgenden Gleichung berechnet:

$$\text{Bekämpfungs-Index (\%)} = \frac{\begin{array}{c}\text{Mittlere Zahl der}\\ \text{Läsionen d. unbe-}\\ \text{handelten Fläche}\end{array} - \begin{array}{c}\text{Mittlere Zahl der}\\ \text{Läsionen d. behan-}\\ \text{delten Fläche}\end{array}}{\begin{array}{c}\text{Mittlere Zahl der Läsionen}\\ \text{der unbehandelten Fläche}\end{array}} \times 100$$

Es wurde gefunden, daß die Verbindung Nr. 4 bei einer Konzentration der aktiven Komponente von 500 ppm eine Bekämpfungswirkung von 100 % zeigte, während die Kontroll-Verbindungen A-1 und B-1 bei 500 ppm einen Bekämpfungs-Wert von 0 % zeigten.

Patentansprüche

1. Tetrahydrochinolin-1-ylcarbonylimidazol-Derivat der allgemeinen Formel (I),

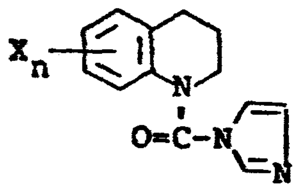

(I)

in der

X ein Chlor-Atom bezeichnet und

n für 0,, 1, 2 oder 3 steht.

2. 1-[8-Chloro-1(H),2,3,4-tetrahydrochinolin-1-ylcarbonyl]imidazol mit der nachstehenden Formel

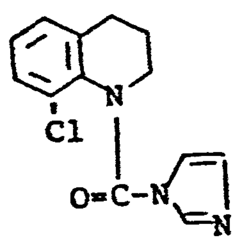

3. 1-[6,8-Dichloro-1(H),2,3,4-tetrahydrochinolin-1-ylcarbonyl]imidazol mit der nachstehenden Formel

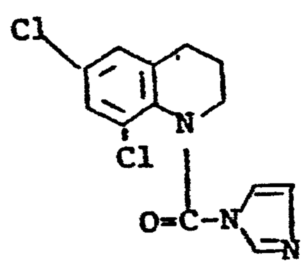

4. Verfahren zur Herstellung eines Tetrahydrochinolin-1-ylcarbonylimidazol-Derivats der allgemeinen Formel (I)

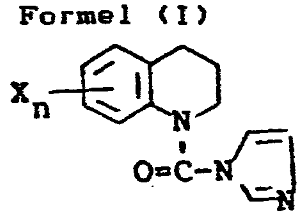

Nit 180

in der

X ein Chlor-Atom bezeichnet und

n für 0, 1, 2 oder 3 steht,

dadurch gekennzeichnet, daß man

i) eine Verbindung der allgemeinen Formel (II),

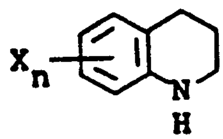

(II)

in der X und n die oben angegebenen Bedeutungen haben, mit einer Verbindung der nachstehenden Formel

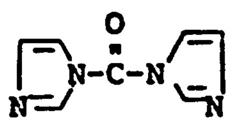

umsetzt,

oder

ii) eine Verbindung der allgemeinen Formel (III)

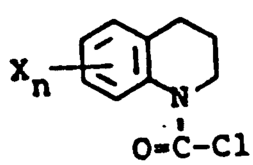

(III)

in der X und n die oben angegebenen Bedeutungen haben, mit Imidazol der nachstehenden Formel

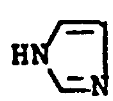

umsetzt.

5. Herbizide oder fungizide Mittel für Landwirtschaft und Gartenbau, gekennzeichnet durch einen Gehalt an mindestens einem Tetrahydrochinolin-1-yl-carbonylimidazol-Derivat der allgemeinen Formel (I)

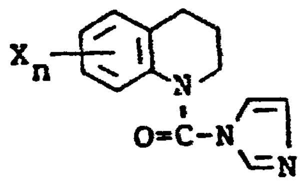

(I)

in der

X ein Chlor-Atom bezeichnet und

n für 0, 1, 2 oder 3 steht.

6. Verwendung des Tetrahydrochinolin-1-ylcarbonylimidazol-Derivates der Formel (I) zur Bekämpfung von unerwünschtem Pflanzenwuchs und zur Bekämpfung von unerwünschten Pilzen.

7. Verfahren zur Herstellung von herbiziden und fungiziden Mitteln, dadurch gekennzeichnet, daß man ein Tetrahydrochinolin-1-ylcarbonylimidazol der Formel (I) mit Streckmitteln und/oder oberflächenaktiven Mitteln vermischt.

8. Verbindung der allgemeinen Formel (III)

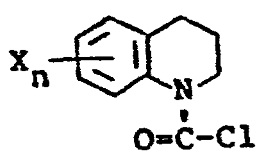

(III)

in der

X ein Chlor-Atom bezeichnet und

n für 0, 1, 2 oder 3 steht.

9. Verfahren zur Herstellung einer Verbindung der allgemeinen Formel (III)

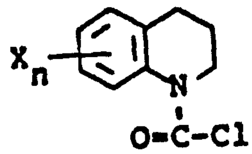

(III)

in der

X ein Chlor-Atom bezeichnet und

n für 0, 1, 2 oder 3 steht,

dadurch gekennzeichnet, daß eine Verbindung der allgemeinen Formel

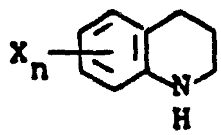

in der X und n die oben angegenbenen Bedeutungen haben, mit Phosgen oder Chlorameisensäure-trichloromethylester umgesetzt wird.